# EUROPEAN PATENT SPECIFICATION

(11) **EP 3 088 036 B1**
(45) Date of publication and mention of the grant of the patent: **20.12.2017**
(21) Application number: 16155233.6
(22) Date of filing: 11.02.2016
(51) Int. Cl.: A61M 25/00, A61M 25/10

(54) **BALLOON CATHETER**
BALLONKATHETER
CATHETER À BALLONNET

(30) Priority: 01.05.2015 JP 2015094088
(43) Date of publication of application: 02.11.2016
(73) Proprietor: ASAHI INTECC CO., LTD., Nagoya-shi, Aichi 463-0024 (JP)
(72) Inventor: SEGI, Kazuteru, Nagoya-shi, Aichi 463-0024 (JP); KATSURADA, Takeharu, Nagoya-shi, Aichi 463-0024 (JP); KUBO, Yuta, Nagoya-shi, Aichi 463-0024 (JP)
(74) Representative: Winter, Brandl, Fürniss, Hübner, Röss, Kaiser, Polte - Partnerschaft mbB

(56) References cited:
- EP-A1- 1 787 673
- EP-A1- 2 859 908
- WO-A1-92/17236
- WO-A1-2014/141440
- JP-A- 2013 106 798

## Description

### TECHNICAL FIELD

The present invention relates to a balloon catheter to be inserted into a stenosis site inside a blood vessel for expanding the stenosis site.

### BACKGROUND ART

Balloon catheters to be inserted into a stenosis site inside a blood vessel for expanding the stenosis site have already been proposed. A balloon catheter mainly comprises a balloon as an expanding body, an outer shaft joined to the proximal end of the balloon and an inner shaft inserted between the balloon and the outer shaft.

Further, it is already known that a wire reinforcement member is provided inside the outer shaft. This reinforcement member is intended to prevent the balloon catheter from kinking in the inside of the blood vessel and providing improved maneuverability when an operator such as a physician inserts a balloon catheter into a blood vessel.

For example, Patent Literature 1 discloses a balloon catheter comprising a wire member (a reinforcement member) inserted through an interior space of a middle shaft where a distal end side thereof is inserted through a fixing tube provided at an outer wall of a guide wire tube or an inner wall of a distal end shaft, and a proximal end side thereof is fixed to a proximal end shaft.

### CITATION LIST

### Patent Literature

Patent Literature 1: Japanese Patent Application Laid-Open No. 2013-106798

### SUMMARY OF THE INVENTION

### TECHNICAL PROBLEM

However, there is a disadvantage in the balloon catheter according to the above Patent Literature 1. The distal end and proximal end of the wire member as a reinforcement member are fixed with the fixing tube and the proximal end shaft, respectively. Therefore, when the balloon catheter is inserted into a curved blood vessel, the wire member as a reinforcement member cannot follow the curvature of the blood vessel smoothly and appropriately, resulting in being bent such that the balloon catheter cannot be further pushed.

Accordingly, an object of the present invention is to provide a balloon catheter in which bending due to a reinforcement member incapable of following the curvature of a blood vessel can be prevented, allowing smooth passage through the curvature of a blood vessel.

### SOLUTION TO PROBLEM

The present invention provides a balloon catheter comprising a balloon, an outer shaft joined to a proximal end of the balloon, an inner shaft inserted into the outer shaft and joined to a distal end of the balloon, and a reinforcement member inserted between the outer shaft and the inner shaft, wherein the inner shaft has a proximal step and a distal step located distally relative to the proximal step, an outer diameter of the proximal step and an outer diameter of the distal step are larger than an outer diameter of the inner shaft positioned between the proximal step and the distal step, and the reinforcement member has a bulged portion capable of moving in an axial direction between the proximal step and the distal step to make contact with at least one of the proximal step and the distal step.

In this configuration, the bulged portion provided at the reinforcement member may move between the proximal step and the distal step in the axial direction. Therefore, the degree of freedom of the reinforcement member will not be lost. As a result, even in a case where the balloon catheter is curved, the reinforcement member will not be forcibly restrained, and for example, a reduced risk of bending in the middle can be reduced. In addition, the range of motion of the reinforcement member is restricted since the bulged portion is configured to be able to move in the axial direction between the proximal step and the distal step of the inner shaft. Therefore, the maneuverability of the balloon catheter can fully be retained. When an operator further pushes the balloon catheter in the direction of the distal end, the bulged portion of the reinforcement member can make contact with the distal step, allowing a pushing force exerted by the operator to be transmitted to the distal end of the balloon catheter. Further, when an operator pulls the balloon catheter in the direction of the proximal end, the bulged portion of the reinforcement member can make contact with the proximal step, allowing the balloon catheter to be quickly brought out.

Further, the following configuration can be considered: the distal step is formed by an end surface of a cylindrical distal large-diameter member attached on the outer periphery of the inner shaft and, the proximal step is formed by an end surface of a cylindrical proximal large-diameter member attached on the outer periphery of the inner shaft.

Further, the following configuration can be considered: the inner shaft has a proximal shaft part formed with a resin, and a distal shaft part that is joined to a distal end of the proximal shaft part and is formed with a resin softer than the resin of the proximal shaft part, and at least one of the proximal step and the distal step is formed so that a proximal end of the distal shaft part covers the distal end of the proximal shaft part, or the distal end of the proximal shaft part covers the proximal end of the distal shaft part.

In this configuration, since the resin hardness of the inner shaft becomes softer toward the distal end, a pushing force can be improved by means of the reinforcement member as well as the inner shaft. Further, since the distal step or the proximal step need not be configured using a separate member, the distal step or the proximal step can easily be formed. By this, the manufacturing process of a balloon catheter can be simplified. In addition, a shaft part formed with a harder resin tends to undergo a so-called kinking more easily. Therefore, the kink resistance at the proximal shaft part can be improved by arranging the reinforcement member between a relatively hard proximal shaft part of the inner shaft and outer shaft part.

Further, in addition to the above configuration, the following configuration is proposed in which the inner shaft has a first shaft part formed with a first resin, a second shaft part formed with a second resin softer than the first resin and joined to the distal end of the first shaft part and a third shaft part formed with a third resin softer than the second resin and joined to the distal end of the second shaft part, and the proximal step is formed so that the proximal end of the second shaft part covers the distal end of the first shaft part, or the distal end of the first shaft part covers the proximal end of the second shaft part, and the distal step is formed so that the proximal end of the third shaft part covers the distal end of the second shaft part, or the distal end of the second shaft part covers the proximal end of the third shaft part.

In this configuration, the resin hardness of the inner shaft becomes softer toward the distal end in a stepwise manner. Therefore, a pushing force can be improved by means of the reinforcement member as well as the inner shaft. Further, since the distal step and the proximal step need not be configured using separate members, both of the distal step and the proximal step can easily be formed. By this, the manufacturing process of a balloon catheter can further be simplified. Again, in this configuration, since the reinforcement member is arranged between the first shaft part of the inner shaft which is relatively hard and the outer shaft part, the kink resistance at the first shaft part can be improved.

Further, a metal wire constituting a coil body is desirably included in at least one of the proximal step and the distal step.

In this configuration, the rigidity of the proximal step or the distal step can be increased by including the metal wire constituting the coil body. Therefore, in a case where the metal wire is included in the distal step, a pushing force when the bulged portion makes contact with the distal step can further be improved. Moreover, in a case where the metal wire is included in the proximal step, the balloon catheter can be brought out more quickly when the bulged portion makes contact with the proximal step.

### ADVANTAGEOUS EFFECT OF THE INVENTION

According to the balloon catheter of the present invention, a reinforcement member is not forcibly restrained when the balloon catheter is inserted into a curved blood vessel. Therefore, a risk of bending in the middle can effectively be reduced, and in addition, the effect of improved maneuverability due to the presence of the reinforcement member can fully be obtained.

### BRIEF DESCRIPTION OF THE DRAWINGS

FIG. 1 shows a top view of a balloon catheter according to Example 1.
FIG. 2 shows a partial enlarged cross-sectional view of the balloon catheter according to Example 1.
FIGS. 3A and 3B show a partial enlarged cross-sectional view of the balloon catheter according to Example 1: FIG. 3A shows a state where the balloon catheter is pushed; FIG. 3B shows a state where the balloon catheter is pulled.
FIG. 4A show a partial enlarged cross-sectional view of the balloon catheter according to Example 2, and FIG. 4B a partial enlarged cross-sectional view of the balloon catheter according to Example 3.
FIG. 5A show a partial enlarged cross-sectional view of the balloon catheter according to Example 4, and FIG. 5B a partial enlarged cross-sectional view of the balloon catheter according to Example 5.
FIG. 6 shows a partial enlarged cross-sectional view of the balloon catheter according to Example 6.
FIG. 7 shows a partial enlarged cross-sectional view of the balloon catheter according to Example 7.
FIG. 8 shows a partial enlarged cross-sectional view of the balloon catheter according to Example 8.

### DESCRIPTION OF EMBODIMENTS

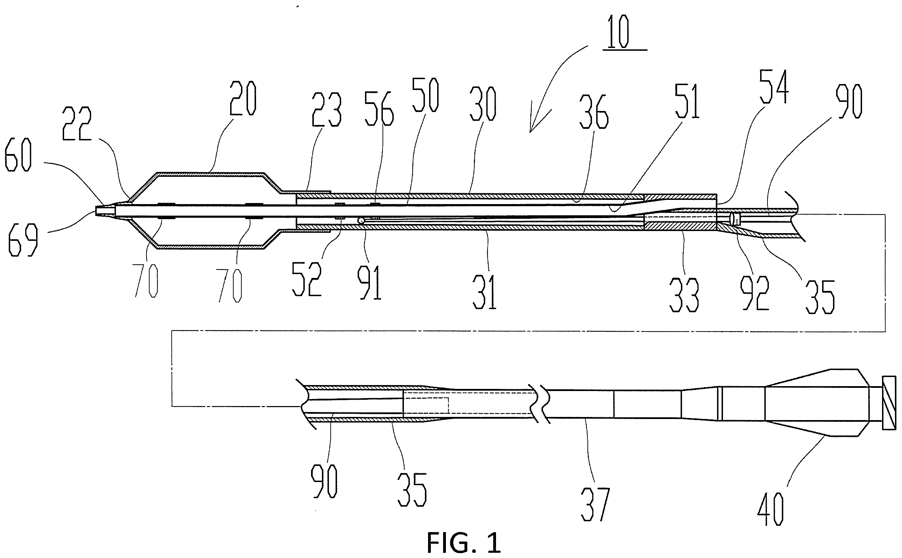

Below, Examples in which the balloon catheters according to the present invention are embodied will be described in detail. However, the present invention shall not be limited to Examples shown below, and modifications in design can appropriately be made. Note that in FIGS. 1 to 8, the left side corresponds to the distal end side (the front end side) which is to be inserted into the body, and the right side corresponds to the proximal end side (the back end side) which is to be operated by an operator such as a physician.

### [Example 1]

As shown in FIG. 1, the balloon catheter 10 used for treating, for example, a stenosis site inside a blood vessel of the heart comprises a balloon 20, an outer shaft 30, a connector 40, an inner shaft 50, a tip 60 and a core wire 90 as a reinforcement member.

The balloon 20 serves to expand a stenosis site, and comprises a member made of a resin. Further, it has a distal end attachment part 22 in the distal end side, and a proximal end attachment part 23 in the proximal end side. The distal end attachment part 22 is joined to the distal end of the inner shaft 50 through the tip 60, and the proximal end attachment part 23 is joined to the distal end of the outer shaft 30.

The outer shaft 30 serves to feed a fluid, and comprises a tubular member which constitutes an inflation lumen 36 for feeding the fluid. Further, the outer shaft 30 has a distal end outer shaft part 31, a guide wire port portion 33, a middle outer shaft part 35 and a proximal end outer shaft part 37 in the order from the distal end side. Note that the guide wire port portion 33 corresponds to a position in which the distal end outer shaft part 31, the middle outer shaft part 35 and the inner shaft 50 are joined.

As further described in detail, the inner shaft 50 is inserted into the distal end outer shaft part 31, and the inflation lumen 36 is formed between the distal end outer shaft part 31 and the inner shaft 50. Moreover, the proximal end outer shaft part 37 comprises a metal tubular member referred to as a so-called hypotube. In addition, the distal end of the proximal end outer shaft part 37 is inserted into and joined to the proximal end of the middle outer shaft part 35. Further, the connector 40 is attached to the proximal end of the proximal end outer shaft part 37. Accordingly, when a liquid for expanding the balloon 20 such as a contrast agent and physiological saline is fed from an indeflator (not shown) attached to the connector 40, the liquid flows into the balloon 20 through the inflation lumen 36, allowing the balloon 20 to expand.

Note that the distal end outer shaft part 31 and the middle outer shaft part 35 are preferably configured to be a tube comprising a resin such as polyamide, polyamide elastomer, polyolefine, polyester, or polyester elastomer. Further, the proximal end outer shaft part 37 is preferably configured with a superelastic alloy such as stainless steel (SUS304) and aNi-Ti alloy.

The inner shaft 50 forms a guide wire lumen 51 for inserting a guide wire (not shown) into the inside. Further, the proximal end of the inner shaft 50 forms a proximal end side guide wire port 54 by being joined to the guide wire port portion 33 of the outer shaft 30. Furthermore, a distal end side guide wire port 69 is formed at the tip 60 arranged at the distal end of the inner shaft 50.

Further, two marker members 70 having a cylindrical shape at each of the distal and proximal sides are arranged on the outer periphery of the inner shaft 50 in the inside of the balloon 20.

The inner shaft 50 is preferably configured with a resin such as polyethylene, polyurethane, polyamide, polyamide elastomer, polyolefine, polyester or polyester elastomer. The tip 60 is preferably formed with a soft resin such as polyurethane, polyurethane elastomer. Moreover, the marker members 70 are preferably configured with a radiopaque metal material such as platinum and tungsten.

Moreover, the core wire 90 is attached to the inner periphery of the distal end of the proximal end outer shaft part 37. The core wire 90 has a circular cross-section, and configured with a tapered metal wire material with a diameter decreased toward the distal end. In addition, the core wire 90 passes through the middle outer shaft part 35 and the guide wire port portion 33, and is formed to extend through the distal end outer shaft part 31. Further, the core wire 90 has a pressing member 92 near the proximal end of the guide wire port portion 33. By this, when a pushing force and a rotating force are applied to the core wire 90, the pressing member 92 makes contact with the guide wire port portion 33, allowing the pushing force and the rotating force to be transmitted to the distal end outer shaft part 31 and the inner shaft 50.

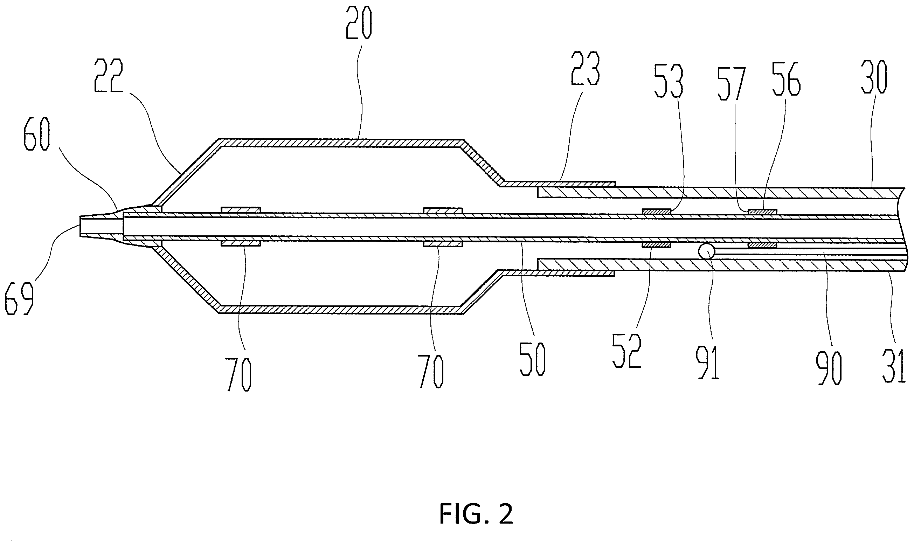

Further, as shown in Fig. 2, on the outer periphery of the inner shaft 50 in the proximal end side relative to the balloon 20, attached are a cylindrical distal large-diameter member 52 and a cylindrical proximal large-diameter member 56 arranged in the proximal end side relative to the distal large-diameter member 52 and spaced from the distal large-diameter member 52.

Further, a distal step 53 is formed by a radial step part formed between the proximal end surface of the distal large-diameter member 52 and the outer periphery of the inner shaft 50. Moreover, a proximal step 57 is formed by a radial step part formed between the proximal end surface of the proximal large-diameter member 56 and the outer periphery of the inner shaft 50. In this configuration, the radical height or outer diameter of the distal step 53 and the radical height or outer diameter of the proximal step 57 are larger than the outer diameter of the inner shaft 50 positioned between the distal step 53 and the proximal step 57.

Note that as in the inner shaft 50, the distal large-diameter member 52 and the proximal large-diameter member 56 are preferably configured with a resin such as polyethylene, polyurethane, polyamide, polyamide elastomer, polyolefine, polyester, or polyester elastomer.

Further, a spherical bulged portion 91 is provided at the distal end of the core wire 90. Moreover, the bulged portion 91 is arranged to be able to move in the axial direction between the distal step 53 and the proximal step 57 provided at the inner shaft 50. Note that the bulged portion 91 may not be necessarily provided at the distal end of the core wire 90, and may be provided at an intermediate position in the core wire 90 although it is preferably provided at the distal end of the core wire 90 in view of the simplicity of the structure.

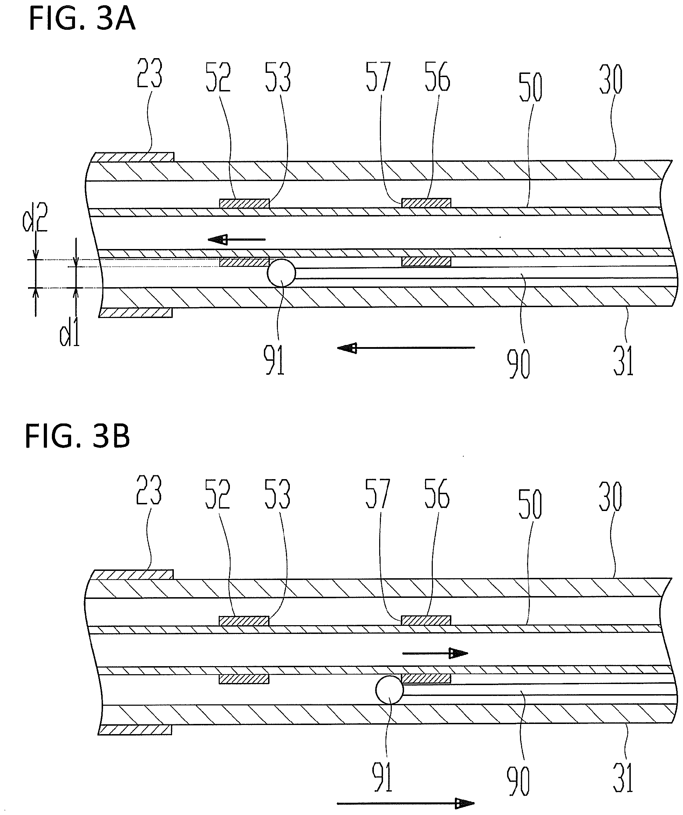

As further described in detail, as shown in FIG. 3A, the minimum space (distance) d1 between the outer peripheries of the steps 53, 57 of the inner shaft 50 and the inner periphery of the outer shaft 30 is set to be smaller in dimension than an outer diameter d2 at the bulged portion 91 of the core wire 90 (dl < d2). Therefore, the bulged portion 91 is capable of moving between the distal step 53 and the proximal step 57 to make contact with both the distal step 53 and the proximal step 57.

Accordingly, as shown in FIG. 3A, for example, when the balloon catheter 10 is pushed, the bulged portion 91 of the core wire 90 makes contact with the distal step 53, allowing the core wire 90 to push the inner shaft 50, and the pushing force applied to the balloon catheter 10 can be transmitted to the distal end part of the balloon catheter 10 without losing it.

Moreover, as shown in FIG. 3B, for example, when the balloon catheter 10 is pulled back, the bulged portion 91 makes contact with the proximal step 57, allowing the core wire 90 to pull the inner shaft 50 and allowing the balloon catheter 10 to be brought out quickly.

As described above, the bulged portion 91 as a reinforcement member provided at the distal end of the core wire 90 is not fixed to the outer shaft 30 or the inner shaft 50 in the balloon catheter 10 according to this Example. Therefore, the degree of freedom of the core wire 90 is retained. Therefore, when the balloon catheter 10 passes through the inside of a curved blood vessel, a risk of bending of the core wire 90 can be reduced. In addition, the excessive movement of the core wire 90 within the outer shaft 30 is suppressed since the range of movement of the core wire 90 in the axial direction is restricted. Therefore, the maneuverability will not be impaired.

Note that according to the present invention, the bulged portion 91 is preferably capable of making contact with both the distal step 53 and the proximal step 57 as described above, but it may be sufficient to be capable of making contact with at least one of the distal step 53 and the proximal step 57.

### [Example 2]

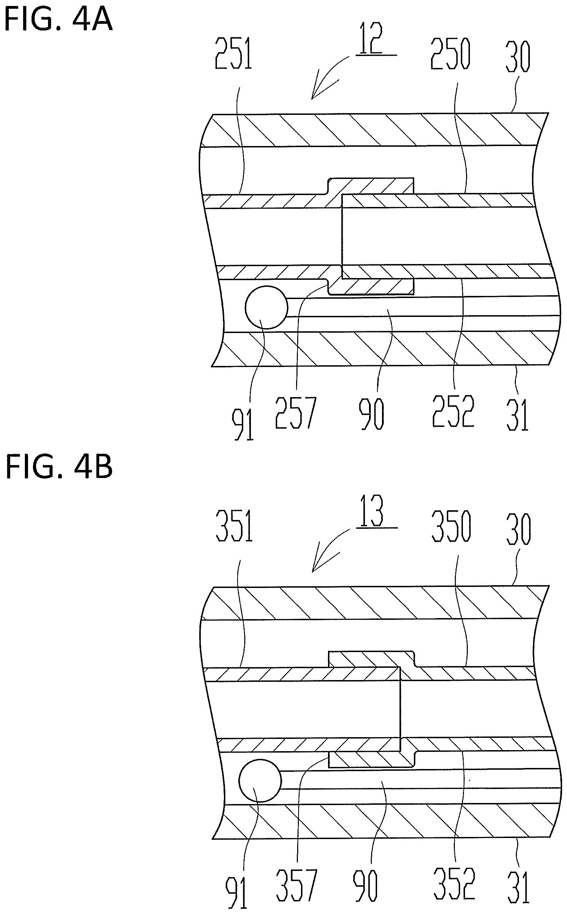

A balloon catheter 12 of Example 2 will be described with reference to FIG. 4A. Note that the same reference number is assigned to those having a similar configuration as in Example 1, and descriptions are omitted.

As shown in Fig. 4A, an inner shaft 250 has a proximal shaft part 252 and a distal shaft part 251 joined to the distal end of the proximal shaft part 252. Note that the proximal shaft part 252 and the distal shaft part 251 are preferably configured to be tubular bodies having an inner diameter and outer diameter substantially equal to each other.

Moreover, the distal shaft part 251 is formed with a resin softer than a resin with which the proximal end side shaft 252 is formed.

Further, the distal end of the proximal shaft part 252 is inserted into the proximal end of the distal shaft part 251, and the proximal end of the distal shaft part 251 covers the distal end of the proximal shaft part 252. By this, a step arises at the distal shaft part 251 to form a proximal step 257.

The resin hardness of the inner shaft 250 becomes softer toward the distal end in the balloon catheter 12. Therefore, a pushing force can be improved by means of the core wire 90 as a reinforcement member as well as the inner shaft 250.

Moreover, it is not necessary to use a separate member to form the proximal step 257. Therefore, the manufacturing process can be simplified. Furthermore, the proximal shaft part 252 formed with a resin harder than the distal shaft part 251 is susceptible to kinking (susceptible to bending). However, the kink resistance of the proximal shaft part 252 can be improved because the core wire 90 is arranged at a position where the proximal shaft part 252 is located.

Further, since the distal end of the proximal shaft part 252 and the proximal end of the distal shaft part 251 overlap to make connection, mutual bonding strength can be increased.

### [Example 3]

A balloon catheter 13 of Example 3 will be described with reference to FIG. 4B. Note that the same reference number is assigned to those having a similar configuration as in Examples 1, 2, and descriptions are omitted.

As shown in FIG. 4B, an inner shaft 350 has a proximal shaft part 352 and a distal shaft part 351 joined to the distal end of the proximal shaft part 352. Note that the proximal shaft part 352 and the distal shaft part 351 are preferably configured to be tubular bodies having an inner diameter and outer diameter substantially equal to each other.

Moreover, the distal shaft part 351 is formed with a resin softer than a resin with which the proximal shaft part 352 is formed.

Further, the proximal end of the distal shaft part 351 is inserted into the distal end of the proximal shaft part 352, and the distal end of the proximal shaft part 352 covers the proximal end of the distal shaft part 351. By this, a step arises between the proximal shaft part 352 and the distal shaft part 351 to form a proximal step 357.

The resin hardness of the inner shaft 350 becomes softer toward the distal end in the balloon catheter 13. Therefore, a pushing force can be improved by means of the core wire 90 as a reinforcement member as well as the inner shaft 350. Further, it is not necessary to use a separate member to form the proximal step 357. Therefore, the manufacturing process can be simplified. Furthermore, the proximal shaft part 352 formed with a resin harder than the distal shaft part 351 is susceptible to kinking. However, the kink resistance of the proximal shaft part 352 can be improved because the core wire 90 is arranged at a position where the proximal shaft part 352 is located.

### [Example 4]

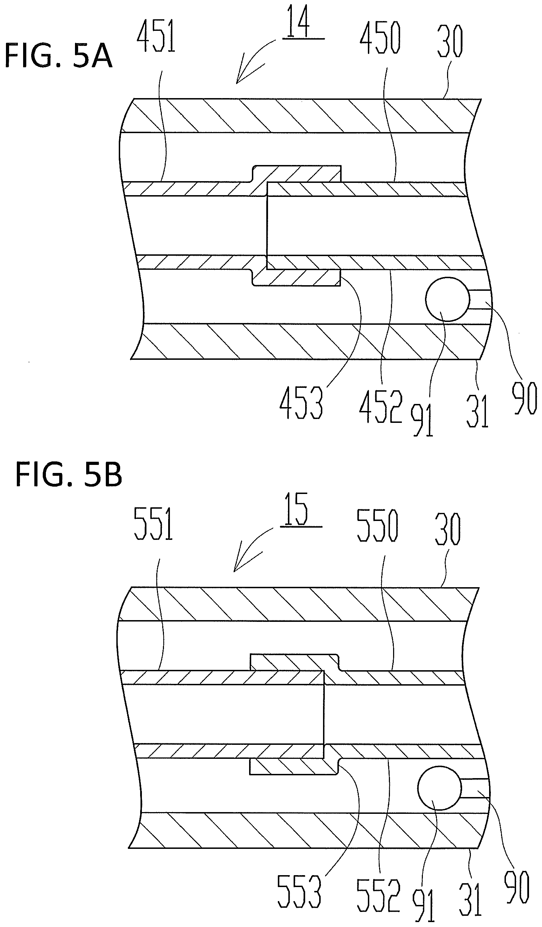

A balloon catheter 14 of Example 4 will be described with reference to FIG. 5A. Note that the same reference number is assigned to those having a similar configuration as in Examples 1 to 3, and descriptions are omitted.

As shown in FIG. 5A, an inner shaft 450 has a proximal shaft part 452 and a distal shaft part 451 joined to the distal end of the proximal shaft part 452. Note that the proximal shaft part 452 and the distal shaft part 451 are preferably configured to be tubular bodies having an inner diameter and outer diameter substantially equal to each other.

Moreover, the distal shaft part 451 is formed with a resin softer than a resin with which the proximal shaft part 452 is formed.

Further, the distal end of the proximal shaft part 452 is inserted into the proximal end of the distal shaft part 451, and the proximal end of the distal shaft part 451 covers the distal end of the proximal shaft part 452. By this, a step arises between the distal shaft part 451 and the proximal shaft part 452 to form a distal step 453.

The resin hardness of the inner shaft 450 becomes softer toward the distal end in the balloon catheter 14. Therefore, a pushing force can be improved by means of the core wire 90 as a reinforcement member as well as the inner shaft 450. Further, it is not necessary to use a separate member to form the distal step 453. Therefore, the manufacturing process can be simplified.

### [Example 5]

A balloon catheter 15 of Example 5 will be described with reference to FIG. 5B. Note that the same reference number is assigned to those having a similar configuration as in Examples 1 to 4, and descriptions are omitted.

As shown in FIG. 5B, an inner shaft 550 has a proximal shaft part 552 and a distal shaft part 551 joined to the distal end of the proximal shaft part 552. Note that the proximal shaft part 552 and the distal shaft part 551 are preferably configured to be tubular bodies having an inner diameter and outer diameter substantially equal to each other.

Moreover, the distal shaft part 551 is formed with a resin softer than a resin with which the proximal shaft part 552 is formed.

Further, the proximal end of the distal shaft part 551 is inserted into the distal end of the proximal shaft part 552, and the distal end of the proximal shaft part 552 covers the proximal end of the distal shaft part 551. By this, a step arises at the proximal shaft part 552 to form a distal step 553.

The resin hardness of the inner shaft 550 becomes softer toward the distal end in the balloon catheter 15. Therefore, a pushing force can be improved by means of the core wire 90 as a reinforcement member as well as the inner shaft 550. Further, it is not necessary to use a separate member to form a distal step 553. Therefore, the manufacturing process can be simplified.

### [Example 6]

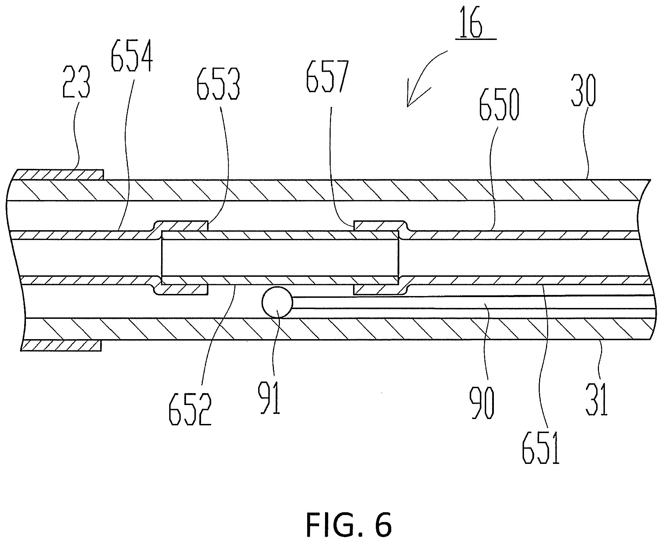

A balloon catheter 16 of Example 6 will be described with reference to FIG. 6. Note that the same reference number is assigned to those having a similar configuration as in Examples 1 to 5, and descriptions are omitted.

As shown in FIG. 6, the inner shaft 650 has a first shaft part 651, a second shaft part 652 joined to the distal end of the first shaft part 651 and a third shaft part 654 joined to the distal end of the second shaft part 652.

The first shaft part 651 is formed with a first resin, and the second shaft part 652 is formed with a second resin softer than the first resin. Further, the third shaft part 654 is formed with a third resin softer than the second resin.

In addition, the distal end of the first shaft part 651 covers the proximal end of the second shaft part 652, and thus a step arises between the first shaft part 651 and the second shaft part 652 to form a proximal step 657 of the inner shaft 650. Further, the proximal end of the third shaft part 654 covers the distal end of the second shaft part 652, and thus a step arises between the third shaft part 654 and the second shaft part 652 to form a distal step 653 of the inner shaft 650.

The resin hardness of the inner shaft 650 becomes softer toward the distal end in the balloon catheter 16. Therefore, a pushing force can be improved by means of the core wire 90 as a reinforcement member as well as the inner shaft 650. Further, it is not necessary to use a separate member to form the distal step 653 and the proximal step 657. Therefore, the manufacturing process can be simplified. Furthermore, the first shaft part 651 formed with a resin harder than the second shaft part 652 and the third shaft part 654 is susceptible to kinking (susceptible to bending). However, the kink resistance of the first shaft part 651 can be improved since the core wire 90 is arranged at a position where the first shaft part 651 is located.

Note that the proximal end of the second shaft part 652 may cover the distal end of the first shaft part 651 to form the proximal step 657, or the distal end of the second shaft part 652 may cover the proximal end of the third shaft part 654 to form the distal step 653.

### [Example 7]

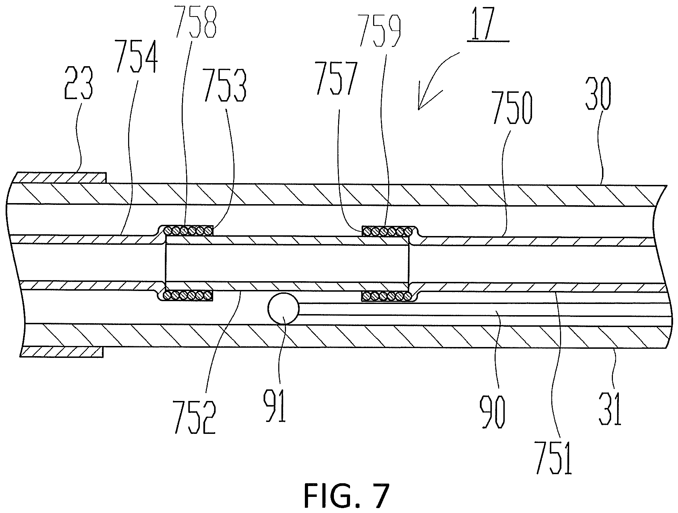

A balloon catheter 17 of Example 7 will be described with reference to FIG. 7. Note that the same reference number is assigned to those having a similar configuration as in Examples 1 to 6, and descriptions are omitted.

As shown in FIG. 7, an inner shaft 750 has a first shaft part 751, a second shaft part 752 joined to the distal end of the first shaft part 751 and a third shaft part 754 joined to the distal end of the second shaft part 752. In addition, a proximal step 757 and a distal step 753 are formed at the inner shaft 750.

Further, a coil body 758 configured with a metal wire is included in the proximal end surface of the third shaft part 754 constituting the distal step 753. Similarly, a coil body 759 configured with a metal wire is also included in the proximal end surface of the first shaft part 751 constituting the proximal step 757.

In the balloon catheter 17, the rigidity of the distal step 753 and the proximal step 757 is improved in addition to the advantages described in the above Example 6. Therefore, when the balloon catheter 17 is pushed, the bulged portion 91 of the core wire 90 makes contact with the distal step 753, and a pushing force exerted by the bulged portion 91 of the core wire 90 can adequately be transmitted to the inner shaft 750, leading to improved maneuverability. Further, when the balloon catheter 17 is pulled back, the bulged portion 91 of the core wire 90 can make contact with the proximal step 757, and a pulling force exerted by the bulged portion 91 of the core wire 90 can adequately be transmitted to the inner shaft 750, allowing the balloon catheters 17 to be brought out quickly.

Note that a coil body may be configured to be included at one of the distal step 753 and the proximal step 757.

### [Example 8]

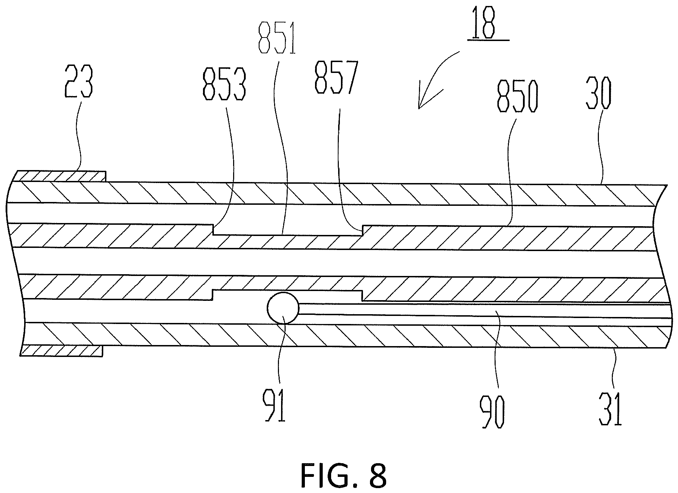

A balloon catheter 18 of Example 8 will be described with reference to FIG. 8. Note that the same reference number is assigned to those having a similar configuration as in Examples 1 to 7, and descriptions are omitted.

As shown in FIG. 8, an inner shaft 850 has a small-diameter portion 851 having a smaller outer diameter as compared with those of other portions. In addition, a distal step 853 is formed at the distal end side of the small-diameter portion 851. Meanwhile, a proximal step 857 is formed at the proximal end side of the small-diameter portion 851.

In the balloon catheter 18, the distal step 853 and the proximal step 857 can be formed by forming the inner shaft 850 with the same outer diameter substantially throughout the full length, and providing the small-diameter portion 851 at a desired position.

Note that the present invention can appropriately be modified other than described in the above Examples 1 to 7. For example, the distal step and the proximal step need not be of a step-like shape as shown in the figures, but may be of a tapered shape as long as the bulged portion 91 of the core wire 90 can freely move in the axial direction within the restriction limit.

Further, the structures of the inner shaft and the outer shaft are not limited to single-layer structures, but may be multiple-layer structures.

Moreover, the shape of the bulged portion 91 is not limited to a globular shape, but may be a cylindrical shape, an elliptic shape and the like.

Note that in the above Examples 1 to 7, the bulged portion 91 is formed by melting the distal end of the core wire 90. However, without limited to these, the bulged portion 91 may be formed (a) by joining a solder material (for example, an Sn-Pb alloy, a Pb-Ag alloy, an Sn-Ag alloy, an Au-Sn alloy and the like), or (b) by joining a hollow coil body configured with a metal wire at the distal end or the middle position of the core wire 90.

Further, for easier understanding, the distal steps 53, 453, 553, 653, 753, 853 and the proximal steps 57, 257, 357, 657, 757, 857 all have the same outer diameter in the above Examples 1 to 7. However, without limited to these, the outer diameter of the distal steps 53, 453, 553, 653, 753, 853 may be different from that of the proximal steps 57, 257, 357, 657, 757, 857. However, the distance between the outer periphery of the distal steps 53, 453, 553, 653, 753, 853 or the proximal steps 57, 257, 357, 657, 757, 857 and the inner periphery of the outer shaft 30 in a radial direction needs to be set smaller than the diameter (the maximum outer diameter) at the bulged portion 91 of the core wire 90 .

Moreover, for easier understanding, a configuration is described where the coil bodies 758, 759 were provided only at the distal step 753 and the proximal step 757 in the above Example 6. However, without limited to this, a coil body may be provided over the full length of the inner shaft 750.

### DESCRIPTION OF SYMBOLS

10 to 18 Balloon catheter
20 Balloon
30 Outer shaft
50 Inner shaft
53 Distal step
57 Proximal step
90 Core wire (Reinforcement member)
91 Bulged portion
250, 350, 450, 550, 650, 750, 850 Inner shaft
251, 351, 451, 551 Distal shaft part
252, 352, 452, 552 Proximal shaft part
257, 357 Proximal step
453, 553 Distal step
651, 751 First shaft part
652, 752 Second shaft part
653, 753, 853 Distal step
654, 754 Third shaft part
657, 757, 857 Proximal step
758, 759 Coil body

## Claims

1. A balloon catheter (10, 12, 13, 14, 15, 16, 17, 18) comprising:
a balloon (20);
an outer shaft (30) joined to a proximal end of the balloon (20);
an inner shaft (50, 250, 350, 450, 550, 650, 750, 850) inserted into the outer shaft (30) and joined to a distal end of the balloon (20);,
a reinforcement member (90) inserted between the outer shaft (30) and the inner shaft (50, 250, 350, 450, 550, 650, 750, 850); and
the inner shaft (50, 250, 350, 450, 550, 650, 750, 850) has a proximal step (57, 257, 357, 657, 757, 857) and a distal step (53, 453, 553, 653, 753, 853) located distally relative to the proximal step (57, 257, 357, 657, 757, 857),
an outer diameter of the proximal step (57, 257, 357, 657, 757, 857) and an outer diameter of the distal step (53, 453, 553, 653, 753, 853) are larger than an outer diameter of the inner shaft (50, 250, 350, 450, 550, 650, 750, 850) positioned between the proximal step (57, 257, 357, 657, 757, 857) and the distal step (53, 453, 553, 653, 753,853), **characterised in that** the reinforcement member (90) has a bulged portion (91) capable of moving in an axial direction between the proximal step (57, 257, 357, 657, 757, 857) and the distal step (53, 453, 553, 653, 753, 853).

2. The balloon catheter (10) according to claim 1, wherein
the distal step (53) is formed by an end surface of a cylindrical distal large-diameter member (52) attached on the outer periphery of the inner shaft (50) and,
the proximal step (57) is formed by an end surface of a cylindrical proximal large-diameter member (56) attached on the outer periphery of the inner shaft (50).

3. The balloon catheter (12, 13, 14, 15, 16, 17) according to claim 1 or 2, wherein hardness of the inner shaft (250, 350, 450, 550, 650, 750) becomes softer toward the distal end.

4. The balloon catheter (12, 13, 14, 15) according to claim 3, wherein the inner shaft (250, 350, 450, 550) comprises a proximal shaft part (252, 352, 452, 552) formed with a resin, and a distal shaft part (251, 351, 451, 551) that is joined to a distal end of the proximal shaft part (252, 352, 452, 552) and is formed with a resin softer than the resin of the proximal shaft part (252, 352, 452, 552), and
wherein at least one of the proximal step (257, 357) and the distal step (453, 553) is formed so that a proximal end of the distal shaft part (251, 451) covers the distal end of the proximal shaft part (252, 452), or the distal end of the proximal shaft part (352, 552) covers the proximal end of the distal shaft part (351, 551).

5. The balloon catheter (16,17) according to claim 3, wherein the inner shaft (657, 757) comprises a first shaft part (651, 751) formed with a first resin, a second shaft part (652, 752) that is joined to a distal end of the first shaft part (651, 751) and is formed with a second resin softer than the first resin, and a third shaft part (654, 754) that is joined to a distal end of the second shaft part (652, 752) and is formed with a third resin softer than the second resin,
the proximal step (657, 757) is formed so that a proximal end of the second shaft part (652, 752) covers the distal end of the first shaft part (651, 751), or the distal end of the first shaft part (651, 751) covers the proximal end of the second shaft part (652, 752), and
the distal step (653, 753) is formed so that a proximal end of the third shaft part (654, 754) covers the distal end of the second shaft part (652, 752), or the distal end of the second shaft part (652, 752) covers the proximal end of the third shaft part (654, 754).

6. The balloon catheter (17) according to any one of claims 1 to 5, wherein a coil body (758, 759) is included in at least one of the proximal step (757) and the distal step (753).

7. The balloon catheter (10, 12, 13, 14, 15, 16, 17, 18) according to any one of claims 1 to 6, wherein an outer diameter (d2) of the bulged portion (91) is larger than a distance (d1) between an outer periphery of the distal step (53, 453, 553, 653, 753, 853) and an inner periphery of the outer shaft (30) in a radial direction, or a distance (d1) between an outer periphery of the proximal step (57, 257, 357, 657, 757, 857) and the inner periphery of the outer shaft (30) in the radial direction.

8. The balloon catheter (10, 12, 13, 14, 15, 16, 17, 18) according to any one of claims 1 to 7, wherein the bulged portion (91) is provided at a distal end of the reinforcement member (90).

## Patentansprüche

1. Ballonkatheter (10, 12, 13, 14, 15, 16, 17, 18) mit:
einem Ballon (20);
einem an ein proximales Ende des Ballons (20) angefügten Außenschaft (30);
einem im Außenschaft (30) angeordneten und an ein distales Ende des Ballons (20) angefügten Innenschaft (50, 250, 350, 450, 550, 650, 750, 850); und
einem zwischen dem Außenschaft (30) und dem Innenschaft (50, 250, 350, 450, 550, 650, 750, 850) angeordneten Versteifungselement (90);
wobei der Innenschaft (50, 250, 350, 450, 550, 650, 750, 850) eine proximale Stufe (57, 257, 357, 657, 757, 857) und eine relativ zur proximalen Stufe (57, 257, 357, 657, 757, 857) distal liegende distale Stufe (53, 453, 553, 653, 753, 853) hat,
**dadurch gekennzeichnet, dass**
der Außendurchmesser der proximalen Stufe (57, 257, 357, 657, 757, 857) und der Außendurchmesser der distalen Stufe (53, 453, 553, 653, 753, 853) größer sind als der Außendurchmesser des Innenschafts (50, 250, 350, 450, 550, 650, 750, 850) zwischen der proximalen Stufe (57, 257, 357, 657, 757, 857) und der distalen Stufe (53, 453, 553, 653, 753, 853), und
das Versteifungselement (90) einen zwischen der proximalen Stufe (57, 257, 357, 657, 757, 857) und der distalen Stufe (53, 453, 553, 653, 753, 853) in axialer Richtung beweglichen ausgebauchten Abschnitt (91) hat.

2. Ballonkatheter (10) nach Anspruch 1, wobei
die distale Stufe (53) von einer Stirnfläche eines im Durchmesser großen zylindrischen distalen Elements (52) gebildet ist, das am Außenumfang des Innenschafts (50) befestigt ist, und
die proximale Stufe (57) von einer Stirnfläche eines im Durchmesser großen zylindrischen proximalen Elements (56) gebildet ist, das am Außenumfang des Innenschafts (50) befestigt ist.

3. Ballonkatheter (12, 13, 14, 15, 16, 17) nach Anspruch 1 oder 2, wobei die Härte des Innenschafts (250, 350, 450, 550, 650, 750) zum distalen Ende hin abnimmt.

4. Ballonkatheter (12, 13, 14, 15) nach Anspruch 3, wobei der Innenschaft (250, 350, 450, 550) einen proximalen Schaftteil (252, 352, 452, 552), der aus einem Harz geformt ist, und einen an das distale Ende des proximalen Schaftteils (252, 352, 452, 552) angefügten distalen Schaftteil (251, 351, 451, 551) aufweist, der aus einem Harz geformt ist, das weicher ist als das Harz des proximalen Schaftteils (252, 352, 452, 552), und
die proximale Stufe (257, 357) und/oder die distale Stufe (453, 553) so ausgebildet ist, dass das proximale Ende des distalen Schaftteils (251, 451) das distale Ende des proximalen Schaftteils (252, 452) oder das distale Ende des proximalen Schaftteils (352, 552) das proximale Ende des distalen Schaftteils (351, 551) ummantelt.

5. Ballonkatheter (16, 17) nach Anspruch 3, wobei
der Innenschaft (657, 757) einen ersten Schaftteil (651, 751), der aus einem ersten Harz geformt ist, einen an das distale Ende des ersten Schaftteils (651, 751) angefügten zweiten Schaftteil (652, 752), der aus eine zweiten Harz geformt ist, das weicher ist als das erste Harz, und einen an das distale Ende des zweiten Schaftteils (652, 752) angefügten dritten Schaftteil (654, 754) aufweist, der aus einem dritten Harz geformt ist, das weicher ist als das zweite Harz,
die proximale Stufe (657, 757) so ausgebildet ist, dass das proximale Ende des zweiten Schaftteils (652, 752) das distale Ende des ersten Schaftteils (651, 751) oder das distale Ende des ersten Schaftteils (651, 751) das proximale Ende des zweiten Schaftteils (652, 752) ummantelt, und
die distale Stufe (653, 753) so ausgebildet ist, dass das proximale Ende des dritten Schaftteils (654, 754) das distale Ende des zweiten Schaftteils (652, 752) oder das distale Ende des zweiten Schaftteils (652, 752) das proximale Ende des dritten Schaftteils (654, 754) ummantelt.

6. Ballonkatheter (17) nach einem der Ansprüche 1 bis 5, wobei in der proximalen Stufe (757) und/oder der distalen Stufe (753) eine Wicklungskörper (758, 759) aufgenommen ist.

7. Ballonkatheter (10, 12, 13, 14, 15, 16, 17, 18) nach einem der Ansprüche 1 bis 6, wobei der Außendurchmesser (d2) des ausgebauchten Abschnitts (91) größer ist als der radiale Abstand (d1) zwischen dem Außenumfang der distalen Stufe (53, 453, 553, 653, 753, 853) und dem Innenumfang des Außenschafts (30) oder der radiale Abstand (d1) zwischen dem Außenumfang der proximalen Stufe (57, 257, 357, 657, 757, 857) und dem Innenumfang des Außenschafts (30).

8. Ballonkatheter (10, 12, 13, 14, 15, 16, 17, 18) nach einem der Ansprüche 1 bis 7, wobei der ausgebauchte Abschnitt (91) an dem distalen Ende des Versteifungselements (90) vorgesehen ist.

## Revendications

1. Cathéter à ballonnet (10, 12, 13, 14, 15, 16, 17, 18) comprenant :
un ballonnet (20) ;
une tige extérieure (30) reliée à une extrémité proximale du ballonnet (20) ;
une tige intérieure (50, 250, 350, 450, 550, 650, 750, 850) insérée dans la tige extérieure (30) et reliée à une extrémité distale du ballonnet (20) ;
un organe de renfort (90) inséré entre la tige extérieure (30) et la tige intérieure (50, 250, 350, 450, 550, 650, 750, 850) ; et
la tige intérieure (50, 250, 350, 450, 550, 650, 750, 850) a un décrochement proximal (57, 257, 357, 657, 757, 857) et un décrochement distal (53, 453, 553, 653, 753, 853) situé distalement par rapport au décrochement proximal (57, 257, 357, 657, 757, 857),
un diamètre extérieur du décrochement proximal (57, 257, 357, 657, 757, 857) et un diamètre extérieur du décrochement distal (53, 453, 553, 653, 753, 853) sont plus grands qu'un diamètre extérieur de la tige intérieure (50, 250, 350, 450, 550, 650, 750, 850) positionnée entre le décrochement proximal (57, 257, 357, 657, 757, 857) et le décrochement distal (53, 453, 553, 653, 753, 853), **caractérisé en ce que**
l'organe de renfort (90) comporte une portion renflée (91) capable de se déplacer dans une direction axiale entre le décrochement proximal (57, 257, 357, 657, 757, 857) et le décrochement distal (53, 453, 553, 653, 753, 853).

2. Cathéter à ballonnet (10) selon la revendication 1, dans lequel
le décrochement distal (53) est formé par une surface d'extrémité d'un organe de grand diamètre distal cylindrique (52) fixé sur la périphérie extérieure de la tige intérieure (50) et,
le décrochement proximal (57) est formé par une surface d'extrémité d'un organe de grand diamètre proximal cylindrique (56) fixé sur la périphérie extérieure de la tige intérieure (50).

3. Cathéter à ballonnet (12, 13, 14, 15, 16, 17) selon la revendication 1 ou 2, dans lequel une dureté de la tige intérieure (250, 350, 450, 550, 650, 750) s'assouplit vers l'extrémité distale.

4. Cathéter à ballonnet (12, 13, 14, 15) selon la revendication 3, dans lequel la tige intérieure (250, 350, 450, 550) comprend une partie de tige proximale (252, 352, 452, 552) formée avec une résine, et une partie de tige distale (251, 351, 451, 551) qui est reliée à une extrémité distale de la partie de tige proximale (252, 352, 452, 552) et est formée avec une résine plus souple que la résine de la partie de tige proximale (252, 352, 452, 552), et
dans lequel au moins l'un du décrochement proximal (257, 357) et du décrochement distal (453, 553) est formé de sorte qu'une extrémité proximale de la partie de tige distale (251, 451) couvre l'extrémité distale de la partie de tige proximale (252, 452), ou l'extrémité distale de la partie de tige proximale (352, 552) couvre l'extrémité proximale de la partie de tige distale (351, 551).

5. Cathéter à ballonnet (16, 17) selon la revendication 3, dans lequel la tige intérieure (657, 757) comprend une première partie de tige (651, 751) formée avec une première résine, et une deuxième partie de tige (652, 752) qui est reliée à une extrémité distale de la première partie de tige (651, 751) et est formée avec une deuxième résine plus souple que la première résine, et une troisième partie de tige (654, 754) qui est reliée à une extrémité distale de la deuxième partie de tige (652, 752) et est formée avec une troisième résine plus souple que la deuxième résine,
le décrochement proximal (657, 757) est formé de sorte qu'une extrémité proximale de la deuxième partie de tige (652, 752) couvre l'extrémité distale de la première partie de tige (651, 751), ou l'extrémité distale de la première partie de tige (651, 751) couvre l'extrémité proximale de la deuxième partie de tige (652, 752), et
le décrochement distal (653, 753) est formé de sorte qu'une extrémité proximale de la troisième partie de tige (654, 754) couvre l'extrémité distale de la deuxième partie de tige (652, 752), ou l'extrémité distale de la deuxième partie de tige (652, 752) couvre l'extrémité proximale de la troisième partie de tige (654, 754).

6. Cathéter à ballonnet (17) selon l'une quelconque des revendications 1 à 5, dans lequel un corps de bobine (758, 759) est inclus dans au moins l'un du décrochement proximal (757) et du décrochement distal (753).

7. Cathéter à ballonnet (10, 12, 13, 14, 15, 16, 17, 18) selon l'une quelconque des revendications 1 à 6, dans lequel un diamètre extérieur (d2) de la portion renflée (91) est plus grand qu'une distance (d1) entre une périphérie extérieure du décrochement distal (53, 453, 553, 653, 753, 853) et une périphérie intérieure de la tige extérieure (30) dans une direction radiale, ou une distance (d1) entre une périphérie extérieure du décrochement proximal (57, 257, 357, 657, 757, 857) et la périphérie intérieure de la tige extérieure (30) dans la direction radiale.

8. Cathéter à ballonnet (10, 12, 13, 14, 15, 16, 17, 18) selon l'une quelconque des revendications 1 à 7, dans lequel la portion renflée (91) est prévue au niveau d'une extrémité distale de l'organe de renfort (90).
